# EUROPEAN PATENT APPLICATION

(11) **EP 3 482 685 A1**
(43) Date of publication of application: **15.05.2019**
(21) Application number: 17200765.0
(22) Date of filing: 09.11.2017
(51) Int. Cl.: A61B 5/06, A61B 5/00, A61B 5/0215, A61B 34/20

(54) **ACTIVELY TRACKED PULMONARY ARTERY CATHETERIZATION**

(71) Applicant: Koninklijke Philips N.V., 5656 AE Eindhoven (NL)
(72) Inventor: Weiss, Steffen, 5656 AE Eindhoven (NL)
(74) Representative: de Haan, Poul Erik

(57) **Abstract**

The present invention relates to an apparatus for assisting catheterization of a heart (10) of a subject (8) comprising a segmented model providing unit (220) for providing a segmented model comprising a plurality of segments (401, 402, 403, 404, 405), wherein each segment corresponds to a predefined region of the heart (10), a segment determination unit (230) for determining a current segment corresponding to the location of an actively tracked pulmonary artery catheter (100), a hemodynamic data providing unit (240) for providing hemodynamic data corresponding to the location of the actively tracked pulmonary artery catheter (100), and an evaluation unit (250) for evaluating the hemodynamic data in reaction to the determined segment. The pulmonary artery catheterization assistance apparatus as well as the corresponding pulmonary artery catheterization assistance method and computer program allow for an improved evaluation of hemodynamic data in pulmonary artery catheterization.

## Description

### FIELD OF THE INVENTION

The present invention relates to the field of pulmonary artery catheterization and in particular to a pulmonary artery catheterization assistance apparatus, a corresponding pulmonary artery catheterization assistance method and computer program. The invention further relates to a joint magnetic resonance imaging apparatus, a pulmonary artery catheter, and a pulmonary artery catheterization system. While the invention is particularly beneficial for right heart catheterizations, its application to further procedures is not limited.

### BACKGROUND OF THE INVENTION

Right heart catheterizations with pulmonary artery catheters (PAC) that can measure the local intravascular blood pressure and blood oxygenation are routinely performed under fluoroscopic guidance, frequently in patients or subjects with congenital heart disease and/or pulmonary hypertension.

In order to accurately evaluate, for instance, the measured blood pressure, a knowledge of the position of a tip portion of the PAC within the subject is required, i.e. the location the blood pressure is acquired has to be correlated to an anatomical location. Known approaches for effectively evaluating the blood pressure, i.e. hemodynamic data, suffer from several drawbacks, for instance the position being difficult to determine accurately, requiring manual effort of a physician and/or implying an undesired dose of ionizing radiation.

### SUMMARY OF THE INVENTION

It has therefore been an object of the present invention to provide a pulmonary artery catheterization assistance apparatus, a corresponding pulmonary artery catheterization assistance method and computer program as well as a joint magnetic resonance imaging apparatus, a pulmonary artery catheter, and a pulmonary artery catheterization system, which allow for an improved evaluation of hemodynamic data in pulmonary artery catheterization.

According to a first aspect, a pulmonary artery catheterization assistance apparatus for assisting catheterization of a heart of a subject is provided. The pulmonary artery catheterization assistance apparatus comprises:
- a location data providing unit for providing location data of an actively tracked pulmonary artery catheter, wherein the location data is indicative of a position of the actively tracked pulmonary artery catheter (PAC),
- a segmented model providing unit for providing a segmented model of the heart of the subject comprising a plurality of segments, wherein each segment corresponds to a predefined region of the heart,
- a segment determination unit for determining a current segment of the plurality of segments of the segmented model using the location data, wherein the current segment corresponds to the location of the actively tracked PAC,
- a hemodynamic data providing unit for providing hemodynamic data corresponding to the location of the actively tracked PAC, and
- an evaluation unit for evaluating the hemodynamic data in reaction to the determined segment.

By knowing the current segment, in which the actively tracked PAC currently resides, the evaluation unit can advantageously perform a segment specific evaluation, which can consider the local differences of the hemodynamic data among the segments, such that an improved evaluation can be performed. Preferentially, the evaluation unit can be configured to determining whether hemodynamic data provided by the hemodynamic data providing unit is as expected for a specific segment, e.g. to differentiate whether the hemodynamic data shows symptoms of pathology or not.

Since the current segment is determined based on location data being indicative of a position of the PAC, the PAC itself is capable of providing location information, so that no undesired external ionizing radiation or manual effort is necessary.

Preferentially, the location data providing unit is configured to provide location data corresponding to local magnetic resonance (MR) responses at the PAC. Therefore, a MR imaging system external to the subject provides MR imaging fields, in particular a gradient field, covering at least the heart of the subject, so that the location data providing unit can provide MR response data indicative of the PAC position. More precisely will the received frequency spectrum be indicative of a position within the applied gradient magnetic field. The location data providing unit can thus preferentially be configured to determine a spatial position based on a received MR response.

The location data providing unit can be a location data receiving unit for receiving location data directly from an actively tracked PAC. For instance, the location data providing unit can in this embodiment be connected directly to a MR conditional wire being connected to a micro-response coil attached at the chip region of a PAC. In other embodiments, the location data providing unit can also be a storing unit, in which previously acquired location data is stored. The location data providing unit is then configured to provide the previously stored location data.

The segmented model providing unit can be configured to provide a previously determined segmented model of the heart. The segmented model of the heart not necessarily covers the entire heart, in some embodiments also only parts of the heart, such as the right atrium and right ventricle, can be provided.

In an embodiment the pulmonary artery catheterization assistance apparatus further comprises a magnetic resonance data providing unit for providing magnetic resonance data of at least the heart of the subject. The MR data providing unit can be a storing unit in which the MR data is stored or a MR data receiving unit for receiving, in particular in real time, MR data from an MR imaging (MRI) apparatus.

The segment determination unit is configured to determine which of the segments provided by the segmented model providing unit most accurately corresponds to the location of the PAC indicated by the location data. Thus, in response to the current segment which is determined by the segment determination unit, a current position of the PAC can be indicated.

The hemodynamic data providing unit can be a storing unit in which hemodynamic data previously acquired can be stored, wherein the previously stored hemodynamic data is provided. The hemodynamic data providing unit can also be a hemodynamic data acquisition unit for acquiring hemodynamic data directly from the PAC. It is known that hemodynamic data indicative of a pressure present at a distal tip of a PAC can be acquired by, for instance, a distal lumen comprising a distal opening at the distal part of the PAC and a pressure sensor connected to the proximal port of the distal lumen. However, also other embodiments of hemodynamic data providing units are of course contemplated, which can provide hemodynamic data corresponding to the location of the PAC.

In an embodiment of the pulmonary artery catheterization assistance apparatus, the segmented model providing unit is configured to determine the segmented model in reaction to MR imaging data of the subject.

In particular, the segmented model providing unit can be configured to receive MR imaging data from the MR data providing unit acquired before a catheterization procedure. Accordingly, there is no PAC visible in the MR imaging data used to determine the segmented model. For instance, the segmented model can be determined in advance based on a MR imaging obtained prior to the procedure, e.g. a different day. Thus, the segmented model can account for individual sizes and shapes of the subject's heart. This is most important due to the fact that right heart catheterizations are mostly performed on subjects suffering from certain heart disease, e.g. deformations of the heart and the like. To this end, the segmented model providing unit preferentially provides a predetermined, standard model of the heart and is configured to adapt this model to the subject's specific shape of the heart based on the MR imaging data. The adaptation can be fully automated or partly automated, wherein an operator such as a physician can be considered to validate and/or improve the segmentation.

In an embodiment of the pulmonary artery catheterization assistance apparatus, the segmented model providing unit is configured to provide each segment of the segmented model as a mesh of triangles.

Since each segment is in this embodiment provided as a mash of triangles, a number of mashes is provided corresponding to the number of individual segments. Mashes of triangles are favorable to process in order to derive, for instance, geometric parameters of the mash, wherein of course also other representations of the segments are contemplated.

In an embodiment of the pulmonary artery catheterization assistance apparatus, the segment determination unit is configured to determine a mean position of each segment of the segmented model and to determine the current segment as the segment with the corresponding mean position having the lowest distance to the location of the actively tracked PAC.

Preferentially, determining the mean position as centre of mass or gravity of the mash can be determined since the segments are coarsely convex. Since accordingly a mean position can be defined for each segment, a distance from the location of the actively tracked PAC can easily be calculated. In this embodiment, it should be noted that the segmented model providing unit preferentially provides the segmented model aligned with the heart of the subject, i.e. in the MR imaging space the location data is also provided in. Expressed differently, the segmented model of the heart and the location of the PAC can directly be compared, since both locations or regions are provided in the same coordinate space.

In an embodiment of the pulmonary artery catheterization assistance apparatus, the hemodynamic data providing unit is configured to provide an average of the hemodynamic data over the residence time of the actively tracked PAC in a segment.

In this embodiment, the hemodynamic data providing unit preferentially additionally provides an average of the hemodynamic data clustered over the residence time of the PAC in different segments. The residence time can be defined as the period between the PAC entering a particular segment and the PAC leaving the respective segment again. Due to the anatomy of the heart, the PAC will subsequently pass through various segments of the heart. The average of the hemodynamic data preferentially disregards data originating close to segment transitions, i.e. near the entering or leaving of a particular segment. The average of the hemodynamic data over the residence in a particular segment provides an additional valuable information for the catheterization procedure.

In an embodiment of the pulmonary artery catheterization assistance apparatus, the segmented model providing unit is configured to provide at least segments corresponding to the right atrium, the right ventricle and the pulmonary arteries.

The right atrium, the right ventricle and the pulmonary arteries are, in order, the main chambers or regions of the heart traversed by a PAC. It is of particular benefit to know whether a tip of the PAC is located in the right atrium, the right ventricle or the pulmonary artery to carry out a beneficial evaluation of the provided hemodynamic data. However, in further embodiments, also alternative or additional segments can be provided for regions of the heart or the surrounding cardiovascular system.

In an embodiment of the pulmonary artery catheterization assistance apparatus, the evaluation unit is configured to at least one of:
- average a mean pressure in case the determined segment corresponds to the right atrium,
- detect and average an a and v wave height in case the determined segment corresponds to the right atrium, and
- average systolic and diastolic pressure in case the determined segment corresponds to the right ventricle or the pulmonary artery.

The particular evaluations take into account the physiological nature of the heart and the hemodynamic pressure curves to be expected in the respective segments of the heart. A systolic and diastolic pressure is not to be expected outside the right ventricle or the pulmonary artery, while a mean pressure is mainly of clinical relevance within the right atrium.

Advantageously, the evaluation can be carried out automatically in reaction to the segment being determined as one of the right atrium, the right ventricle or the pulmonary artery. The PAC will pass, in order, through all of these segments and the evaluation unit is preferentially adapted to account for this subsequent change of the segment and adapt the evaluation accordingly. According to this embodiment, the clinically relevant evaluation of the hemodynamic data is readily available for the right atrium, the right ventricle and the pulmonary artery, respectively. A physician can thus directly be presented the relevant evaluated data for the respective segment.

It should be noted that an average preferentially should be understood to include a standard deviation or other statistical measures in addition to the calculated mean value. The mean pressure is preferentially averaged over the entire duration of the persistence of the PAC within the right atrium, while in other embodiments also a certain excerpt of the persistence time can be employed.

The a and v waves are features that are expected to be detectable in the right atrium, wherein the a and v wave can be determined by their characteristics, respectively. For instance, the a wave is expected to be taller and briefer than the v wave, which is expected to be less tall and sustained. In pathological states the differences between a and v wave can get blurred.

Finally, systolic and diastolic pressures can, for instance, be determined as the maximum and minimum hemodynamic pressure obtained during a cardiac cycle. The value of the systolic and diastolic pressure to be expected preferentially differs among the right ventricle and the pulmonary artery. In particular, a higher diastolic value is expected in the pulmonary artery than in the right ventricle. Thus, by determining for instance a diastolic value in reaction to the determined segment, it can preferentially be evaluated whether the diastolic value is in a normal or pathological range and further evaluation has to be carried out.

In an embodiment of the pulmonary artery catheterization assistance apparatus, the pulmonary artery catheterization assistance apparatus further comprises an inflation status determination unit for determining an inflation status of the actively tracked pulmonary artery catheter.

Since the inflation status determination unit is configured to determine the inflation status of the actively tracked PAC, the evaluation of the hemodynamic data can further be improved. More particularly, since the evaluation can be performed in response to whether the balloon is inflated or not, it can be decided whether a pulmonary pressure is a pulmonary wedge pressure, which is measured by wedging a pulmonary artery catheter with an inflated balloon into a branch of the pulmonary artery.

The inflation status determination unit can comprise an inflation status receiving unit for receiving an inflation status of the PAC, e.g. an inflation status providing unit of the PAC. The inflation status determination unit can alternatively or additionally be an inflation status providing unit of the PAC itself, e.g. a pump unit connected with the PAC to achieve inflation and/or deflation of the balloon thereof.

In an embodiment of the pulmonary artery catheterization assistance apparatus, the inflation status determination unit is configured to determine the inflation status in reaction to a volume of liquid injected into a balloon port of the actively tracked pulmonary artery catheter, and to derive the inflation status as a percentage of full balloon inflation based on the volume of liquid injected.

Preferentially, the full balloon inflation volume is known and the percentage of inflation can be derived from the volume of liquid injected into the balloon. To this end, it is known to provide PAC's with a balloon lumen, which leads from a balloon port to the balloon in order to inflate or deflate the balloon.

In case the inflation status determination unit determines that a user defined threshold is exceeded or a certain inflation status is maintained over a specified minimum time, it can be deduced that a pulmonary arterial branch is wedged, i.e. a pulmonary wedge pressure can be provided as the hemodynamic data. The user defined threshold and/or user defined minimum time can be determined generically, i.e. equal for all subjects. However, the respective thresholds can also be provided subject-specific, i.e. adapted to the specific subject. Further, preferentially, the inflation status determination unit can determine the inflation status dependant on the location, i.e. adapt particularly the thresholds depending on the location of the balloon.

In an embodiment of the pulmonary artery catheterization assistance apparatus, the inflation status determination unit is configured to determine the balloon to be inflated in case the inflation status exceeds a user defined threshold or maintains at a certain inflation status over a user defined minimum time.

In an embodiment of the pulmonary artery catheterization assistance apparatus, in case the inflation status determination unit determines the balloon to be inflated, the evaluation unit is configured to at least one of:
- average a mean wedge pressure, and
- detect and average an a and v wave height.
Advantageously, in case it is determined that the balloon is inflated, i.e. a branch of the pulmonary artery is wedged, a pulmonary wedge pressure is present as the hemodynamic data. Accordingly, since the evaluation unit can evaluate the hemodynamic data in response to determining that a pulmonary wedge pressure is provided as the hemodynamic data, more elaborate evaluation of the hemodynamic data can be performed. In this context, as described with reference to the further evaluation, a mean wedge pressure is averaged while at the same time, preferentially, a standard deviation is determined. Further, a and v wave heights can be obtained as for other segments of the heart.

In an embodiment of the pulmonary artery catheterization assistance apparatus, the pulmonary artery catheterization assistance apparatus further comprises a residence time evaluation unit, wherein the residence time evaluation unit is configured to evaluate a residence time of the actively tracked pulmonary artery catheter in a determined segment and to provide an indication to a user in case enough data for the determined segment is evaluated.

The residence time is preferentially defined as the time period between the PAC entering a specific segment and the PAC entering a different segment, i.e. leaving the previously determined segment. In this embodiment, the indication in case enough data is evaluated can advantageously be used, for instance, to point the operator or physician to advance the catheter so that a more efficient treatment in terms of treatment time can be achieved. A shorter treatment time minimizes the risks of complications of the catheterization. Thus, since the treatment time can be reduced, also less dangerous treatment can be performed.

In an embodiment, enough data is present in case the standard deviation of a derived value falls below a certain value. In other examples, enough data is present in case a certain number of heart cycles is evaluated for a specific segment. The examples of enough data are of course not limited and also further examples of a determination of enough data are likewise considered by a skilled person.

In an embodiment of the pulmonary artery catheterization assistance apparatus, the pulmonary artery catheterization assistance system further comprises a display unit for generating a display for assisting a user, the generated display comprising a representation of the segmented model, wherein the segments are displayed with a respective different representation in response to the evaluated hemodynamic data.

The display generated by the display unit can assist the physician or operator during the pulmonary artery catheterization. For instance, a segmented model can be overlaid with a representation of the catheter and/or real time images reconstructed from an MR imaging system. In this embodiment, the display unit preferentially displays the segments with respective different representations, so that the physician or operator intuitively and directly correlates the position of the PAC with the current segment of the heart.

The respective different representations can account for different parameters of the evaluated hemodynamic data. For instance, the representation can include a color coding indicating the mean pressure or systolic pressure or diastolic pressure in the respective chamber. In other examples, the representation can include a color coding indicating whether a specific segment is sufficiently sampled, i.e. sufficient hemodynamic data has been evaluated for the particular segment. In this example, a segment can be represented with, for instance, a green color, in case sufficient data is evaluated, while segments, which still lack data, can be represented with, for instance, red color. In other examples, the respective different representation can be indicative of a pathologic condition of the segment, for instance, a red color can indicate that the specific segment suffers from some kind of pathology, wherein a healthy segment can be depicted in a different color. Additionally or alternatively, a user can also be capable of switching between one or more of these representations.

In an embodiment of the pulmonary artery catheterization assistance apparatus, the pulmonary artery catheterization assistance apparatus further comprises
- a chamber volume determination unit for determining a volume of a chamber of the heart based on provided MR image data, and
- a pressure-volume loop determination unit for determining a pressure-volume loop by acquiring the volume of the chamber of the heart, determined from the chamber volume determination unit, and the hemodynamic data, provided by the hemodynamic data providing unit, at simultaneous times.

Preferentially, the MR-image data is provided by the MR-image data providing unit as described above. The chamber of the heart preferentially corresponds to the current segment the PAC is determined to be located in. Accordingly, the chamber volume determination unit is then preferentially configured to determine the volume of the chamber corresponding to the current segment based on methods known in the art of MRI.

Further, in response thereto, the pressure-volume loop determination unit is configured to determine the pressure-volume loop by simultaneous acquisition of the volume, i.e. in response to the provided MR-image data, and the hemodynamic data obtained at the corresponding location. Thus, chamber volume and pressure can be recorded over one or more loops of the cardiac cycle. Since the location of the PAC is advantageously determined in a particular segment, the pressure-volume loop can be acquired without manual input of the operator in response to an accurate location determination, and can thus be obtained more reliably.

In an embodiment of the pulmonary artery catheterization assistance apparatus, the pressure-volume loop determination unit is configured to initiate a pressure-volume loop acquisition in response to a change in the determined segment.

In this embodiment, the pressure-volume loop determination unit initiates the acquisition of the pressure-volume loop without necessary intervention of the operator, simply by detecting the PAC changing the determined segment, i.e. entering a desired segment. The initiation can occur instantaneously or after expiration of a certain time, for instance, after the completion of one or a plurality of cardiac cycles. According to this embodiment, additional valuable evaluation information, i.e. the pressure-volume loop, can be directly acquired without the operator having to decide on the correct time for initiating the pressure-volume loop acquisition.

In a second aspect of the invention, a magnetic resonance imaging apparatus for use with a pulmonary artery catheterization assistance apparatus according to the first aspect of the present invention is provided. The magnetic resonance imaging apparatus comprises a separate magnetic resonance receiver configured to be connected to an RF wire of an actively tracked PAC.

It is known that MR imaging apparatuses can comprise multiple RF coils, which each measure MR responses of certain tissue, e.g. tissue surrounding the respective coils. Preferentially, in this aspect, a separate MR receiver is provided, which is configured to receive location information based on an RF wire of a tracking component of an actively tracked PAC. The magnetic resonance imaging apparatus according to this aspect further provides valuable MR imaging data of the heart of the subject, which can further be evaluated by, for instance, the segmented model providing unit and/or the magnetic resonance data providing unit of the PAC assistance apparatus according to the invention. Likewise, all advantages as described with the PAC assistance apparatus can be obtained with the MRI apparatus according to this aspect.

In a third aspect of the invention, a pulmonary artery catheter (PAC) for use with a pulmonary artery catheterization assistance apparatus according to the first aspect is provided. The pulmonary artery catheter comprises
- an inflatable balloon arranged at a distal portion of the PAC,
- a distal lumen including a distal lumen opening arranged at a distal portion of the catheter for providing hemodynamic data corresponding to the location of the distal lumen opening, and
- an active tracking component for providing position data indicative of a position of the catheter within a magnetic resonance imaging system.

Since the PAC according to this aspect comprises an active tracking component, the PAC allows to accurately determine the position of the PAC, in particular of a tip including the inflatable balloon thereof. The active tracking component preferentially is configured to provide position data directly to a location data providing unit of the PAC assistance apparatus so that the PAC position can be determined easily. Further, the hemodynamic data being provided in the distal lumen can directly be obtained by the hemodynamic data providing unit of the PAC assistance apparatus, for instance, comprising a pressure sensor or the like at a proximal port of the distal lumen.

In an embodiment of the pulmonary artery catheter, the active tracking component comprises in particular at least one µ-coil and a magnetic resonance conditional wire along the catheter being attached to the µ-coil.

Since the active tracking component comprises a micro coil, magnetic resonance signals of the surrounding tissue can be received by the µ-coil and transmitted via the magnetic resonance conditional wire to the outside of the catheter. The MR conditional wire is connected to one of the receiver of the MRI system, which is a part of the location data providing unit of the PAC. Preferentially, the at least one µ-coil is arranged close to the distal opening of the PAC, while in other embodiments, also further locations of the µ-coil are feasible. It is preferred that the µ-coil be arranged close to the distal portion of the PAC in order to provide accurate location information. In other embodiments, multiple µ-coils can be provided at different locations along the PAC to allow for determination of not only the tip position but of the entire shape of the distal section of the shaft. In this example, the micro coils are preferentially connected with separate MR conditional wires, while also a single common MR conditional wire can be imagined.

In a fourth aspect of the invention, a pulmonary artery catheterization system is provided. The pulmonary artery catheterization system comprises
- an MR imaging apparatus according to the second aspect,
- a PAC according to the third aspect, and
- a pulmonary artery catheterization assistance apparatus according to the first aspect.

In a fifth aspect, the use of an actively tracked catheter in right-heart catheterization is provided.

In a sixth aspect, a pulmonary artery catheterization assistance method for assisting catheterization of a heart of a subject is provided. The pulmonary artery catheterization assistance method comprises:
- providing location data of an actively tracked pulmonary artery catheter, wherein the location data is indicative of a position of the actively tracked pulmonary artery catheter,
- providing a segmented model of the heart of the subject comprising a plurality of segments, wherein each segment corresponds to a predefined region of the heart,
- determining a current segment of the plurality of segments of the segmented model using the location data, wherein the current segment corresponds to the location of the actively tracked pulmonary artery catheter,
- providing hemodynamic data corresponding to the location of the actively tracked pulmonary artery catheter, and
- evaluating the hemodynamic data in reaction to the determined segment.
In a seventh aspect, a computer program for assisting catheterization of a heart of a subject is provided. The computer program comprising program code means for causing a pulmonary artery catheterization system according to the fourth aspect to carry out the steps of the pulmonary artery catheterization assistance method according to the sixth aspect, when the computer program is run on a processing unit of the pulmonary artery catheterization system.

It shall be understood that the pulmonary artery catheterization assistance apparatus according to the first aspect, the magnetic resonance imaging apparatus according to the second aspect, the pulmonary artery catheter according to the third aspect, the pulmonary artery catheterization system according to the fourth aspect, the use of an actively tracked catheter according to the fifth aspect, the pulmonary artery catheterization assistance method according to the sixth aspect and the computer program according to the seventh aspect have similar and/or identical preferred embodiments, in particular, as defined in the dependent claims.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

In the following drawings:
Fig. 1 shows schematically and exemplarily a pulmonary artery catheter (PAC),
Fig. 2 shows schematically and exemplarily a pulmonary artery catheterization assistance system,
Fig. 3 shows schematically and exemplarily illustrates a hemodynamic curve at different positions of the PAC,
Fig. 4 shows schematically and exemplarily a display of a segmented model of the heart, and
Fig. 5 shows schematically and exemplarily a flowchart of a pulmonary artery catheterization assistance method.

### DETAILED DESCRIPTION OF EMBODIMENTS

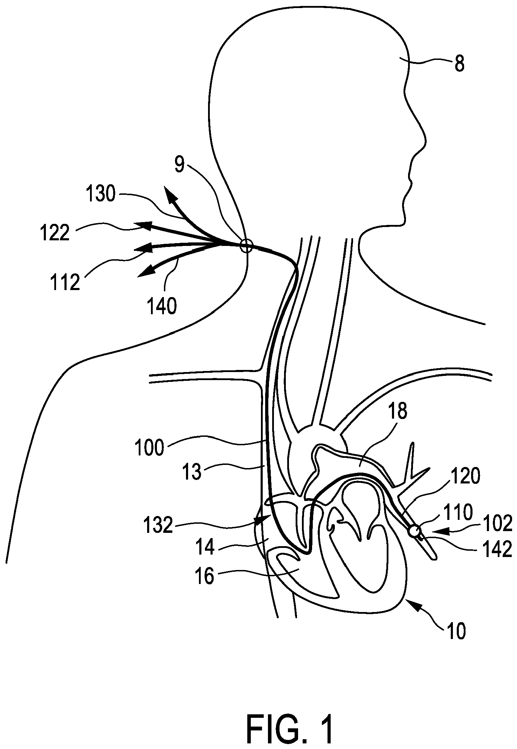

Fig. 1 schematically and exemplarily illustrates a pulmonary artery catheter 100 used in catheterization of a heart 10 of a subject 8. The catheter 100 is inserted into the body of the subject 8 at an exemplary location 9 and advanced through the subject's vascular system passing the superior vena cava 12, right atrium 14, right ventricle 16 until reaching eventually the pulmonary artery 18. Right heart catheterizations with pulmonary artery catheters, such as catheter 100, that can measure the local intravascular blood pressure and blood oxygenation, provide clinically important information on cardiac performance and systemic oxygen transport. Pulmonary artery catheters known in the art are also referred to as Swan-Ganz catheters.

Throughout the right heart catheterization, it is of utmost importance to accurately know the location of the pulmonary artery catheter 100, in particular of a distal tip 102 thereof. For this purpose, classically right heart catheterizations are performed under fluoroscopic guidance, wherein a contrast fluid is for instance received within a balloon 110 located near the distal tip 102 of the catheter 100.

The catheter comprises multiple lumens, wherein the number is four in this example. In connection with the balloon 110 is a balloon port 112, through which an inflation status of the balloon 110 can be controlled. Two further lumens include a proximal port 130 lumen and a distal lumen port 140. The distal lumen port 140 leads into a lumen connecting to a distal lumen opening 142 and the proximal lumen port 130 leads to a proximal lumen opening 132, which is arranged at a certain distance from the distal tip 102.

The distal lumen opening 142 connected to the distal lumen port 140 via a lumen allows for a measurement of the pressure present at the distal tip 102 of the catheter 100. Preferentially, a pressure sensor is connected to the lumen at the distal lumen port 140 thereof as known from conventional pressure catheters. Optionally, also at the proximal lumen port 130 a pressure sensor can be arranged.

As will be detailed in the following, in magnetic resonance (MR) guided catheterization, pressure at the proximal lumen opening 132 is not necessary to be determined. Accordingly, also the provision of the proximal lumen including proximal lumen port 130 and proximal lumen opening 132 can be omitted in MR guided applications.

Since the classically contrast-agent filled balloon requires X-ray fluoroscopy to monitor the position of the distal tip 102, ionizing radiation is required, which is a general concern to avoid or minimize if possible. In an alternative approach, which is followed according to the present invention, real-time MR guidance is used.

However, in contrast to a projection technique, MR guidance must be performed with slices of finite thickness, which causes the problem that the balloon 110 becomes invisible as soon as it leaves the current imaging slice.

For this reason, the pulmonary artery catheter 100 according to this example comprises an active tracking component 120, which comprises at least one micro-receive coil in the region of the distal tip 102 and is connected to a separate MR receiver by a MR conditional RF wire 122. The RF wire 122 is provided, for instance, in one of the lumens of the pulmonary artery catheter 100.

The active tracking component 120 is thus capable of determining the location of the pulmonary artery catheter 100 relative to an externally applied gradient field using magnetic resonances of surrounding matter or tissue. Real-time position monitoring of invasive devices using magnetic resonance is described, for instance, in the article by C. L. Doumlin et al. "Real-time position monitoring of invasive devices using magnetic resonance", Magnetic Resonance in Medicine, 1993, pages 411 to 415.

It is known that wires generally generate heat under the influence of magnetic resonance imaging, in particular during radiofrequency transmission. This can lead to damage to surrounding tissue, which is to be avoided. A wire, which is save to use in an MR system, is described, for instance, in the article by S. Weiss et al. "Transmission Line for Improved RF Safety of Interventional Devices", Magnetic Resonance in Medicine, 2005, pages 182 to 189.

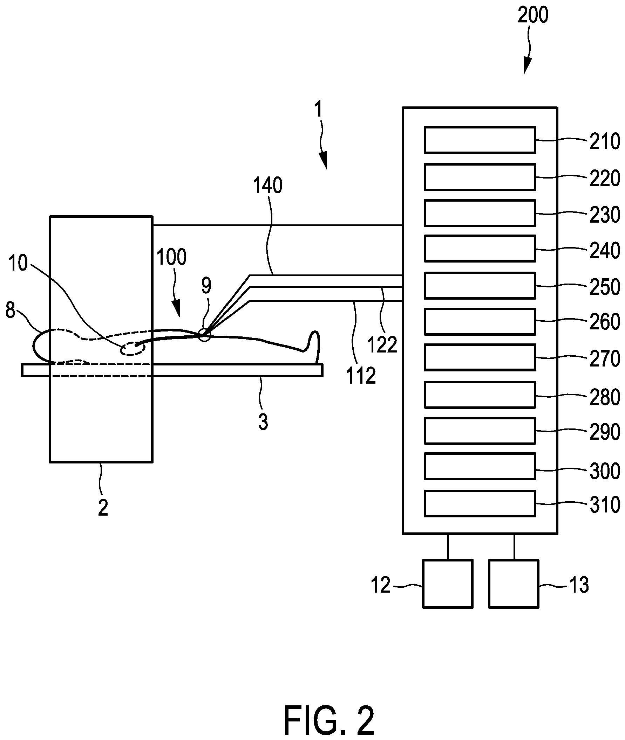

The position of the pulmonary artery catheter 100 can thus be known accurately using the MR response signals of the active tracking component 120. This information can be used advantageously to assist a user, such as a physician, in performing the catheterization. This will be explained in more detail with respect to the pulmonary artery catheterization assistance system exemplified in Fig. 2.

Right heart catheterizations are frequently performed in patients with congenital heart disease and/or pulmonary hypertension. By determining pulmonary artery pressure and flow, the pulmonary vascular resistance (PVR), which is used together with the response of PVR to nitric oxide (NO) and oxygen to assess suitability for surgery and need for vasodilator therapy, as further detailed, for instance, in the article by V. Muthurangu et al. "Novel Method of Quantifying Pulmonary Vascular Resistance by Use of Simultaneous Invasive Pressure Monitoring and Phase-Contrast Magnetic Resonance Flow", Circulation 2004, pages 826 to 834.

Conventional pulmonary artery catheters rely on the principle of thermodilution or the Fick principle to determine flow. In contrast, since the pulmonary artery catheter 100 according to the invention is magnetic resonance conditional, and since further a MR system is used for supplying the necessary excitation for the active tracking component 120, quantitative MR measurements can be used instead for accurately and directly measuring a time dependent flow through vessels, in particular the pulmonary artery. Thus, problems related to thermodilution can be avoided using the pulmonary artery catheter 100 according to the present invention. This is described in more detail, for instance, in the article by W. G. Hundley et al. "Quantitation of cardiac output with velocity-encoded, phase-difference magnetic resonance imaging", The American Journal of Cardiology, 1995, pages 1250 to 1255.

Further information helpful in right heart catheterization can be assessed accurately by fast MR imaging (MRI) with frame rates in the order of 10 Hz as described in the article by W. R. T. Witschey et al. "A Real-Time Magnetic Resonance Imaging Technique for Determining Left Ventricle Pressure-Volume Loops", Ann. Thorac Surg., 2014, pages 1597 to 1603. The time resolved simultaneous measurement of chamber volume and invasive pressure allows, among others, to acquire pressure volume loops (PVL), which are an important diagnostic tool, characterizing cardiac function and pathologies. Further relevant parameters can be derived from volume data including calculation of end-diagnostic volume (EDF), end-systolic volume (ESV), ejection fraction (EF), stroke volume (SV) and cardiac output (CO).

Pulmonary artery catheter 100 is not necessarily actively steered, but comprises balloon 110 at the distal tip 102 thereof, which is driven forward by the blood stream as known in the art. After insertion at an entry point, e.g. location 9, into a peripheral vein, the catheter is washed, as described, through the superior vena cava 12, the right atrium 14 and the ventricle 16 into the pulmonary artery 18. Further advancement is stopped due to the reduced diameter in one of the pulmonary arteries. However, catheters driven by the blood stream can make it difficult to be advanced in a controlled way to various locations within the right heart due to malformations of the heart including shunts, vascular narrowing or valve deformations, particularly in patients with congenital malfunctions of the heart. For this reason, in a further example of the pulmonary artery catheter 100, an MR-conditional active tip deflect mechanism is included in the same way as has been previously described, cf. the article by S. Weiss et al. "MR-guided cardiac radiofrequency ablation with catheter-tracked local MR lesion monitoring", Journal of Cardiovascular Magnetic Resonance, 2013, pages 1 and 2, for a different type of MR conditional catheters, namely an MR conditional RF ablation catheter.

The pulmonary artery catheter 100 will now further be described in connection with a pulmonary artery catheterization system 1 as illustrated in Fig. 2.

Fig. 2 schematically and exemplarily illustrates pulmonary artery catheterization system 1 used for carrying out and assisting a pulmonary artery catheterization of subject 8. Subject 8 is positioned on a support means 3 like a patient table within a magnetic resonance imaging apparatus 2 configured to provide MR data of the heart 10 of the subject 8. Further, MR imaging apparatus 2 is capable of providing a gradient field within the imaging region, so that active tracking component 120 of PAC 100 is capable of providing a location information of PAC 100.

In this example, the data of magnetic resonance imaging apparatus 2 and the PAC 100, more particular RF wire 122 thereof, are provided to a PAC assistance apparatus 200 according to an aspect of the invention, which will be described the following:

PAC assistance apparatus 200 comprises a location data providing unit 210, a segmented model providing unit 220, a segment determination unit 230, a hemodynamic data providing unit 240, an evaluation unit 250, an inflation status determination unit 260, a residence time evaluation unit 270, a display unit 280, a chamber volume determination unit 290, a pressure volume loop determination unit 300 and an MR data receiving unit 310. PAC assistance apparatus 200, which can for instance be implemented or integrated within a processing unit or a computer system of the MR apparatus 2, can further interact with an input unit 12 like a keyboard, a computer mouse, a touchpad, etc., and an output unit 13 like a display. It should be noted that further components of the MR imaging apparatus 2 are not illustrated in this schematic drawing, while these further components, such as a MR apparatus controller, are of course present.

Location data providing unit 210 is configured to provide location data of PAC 100, wherein in this example location data providing unit 210 is configured to receive the MR response of active tracking component 120 transmitted by RF wire 122. Location data providing unit 210 can thus be a separate MR receiver or connected to a dedicated MR receiver of the MR imaging system. The location data provided by RF wire 122 to location data providing unit 210 is indicative of a position of the actively tracked PAC 100, for instance can be translated into a location in coordinates of the MR imaging system.

Segmented model providing unit 220 provides a segmented model of at least some parts of the heart 10 of the subject 8 wherein each segment of the segmented model corresponds to a predefined region or chamber of the heart 10. More precisely, in this example the segmented model comprises a segment of the right atrium, the right ventricle and the pulmonary artery. In other examples, the segmented model can also comprise alternative or additional segments of the heart 10. Segmented model providing unit 220 provides the segmented model based on a generic model of a heart 10, which is adapted to the specific heart of the subject 8 using an MR image of MR imaging apparatus 2 prior to the catheterization procedure. This adaption of the segmented model to the individual heart of the subject 8 is preferentially done at least partly automated. Further, during catheterization, the location of the heart within the imaging space of the MR imaging apparatus 2 is preferentially correlated with the segmented model, so that the location of the tracked PAC 100 is provided in the same coordinate space as the segmented model, such that a position of the PAC 100 can be identified within the subject 8 relative to the segments of the provided segmented model.

To this end, segment determination unit 230 is configured to provide one of the segments of the segmented model as a current segment, in which the PAC 100 currently resides. More precisely, the current segment is the segment of the plurality of segments, in which the distal tip 102 of PAC 100 resides. In this example, segment determination unit 230 is configured to calculate a mean value or a centre of mass of each of the segments and determine the current segment as the segment out of the plurality of segments, which has the lowest distance to the determined position of the PAC 100.

Hemodynamic data providing unit 240 is configured to provide hemodynamic data corresponding to the location of the PAC 100. In this example, hemodynamic data providing unit 240 is configured to receive hemodynamic data from the distal tip 102 of PAC 100 using pressure information, which is measurable at a distal opening 142 thereof. The pressure information is transmitted via a distal port of the distal lumen 140, wherein a pressure sensor for digitizing and converting the hemodynamic data can be integrated into hemodynamic data providing unit 240 or be part of the pulmonary artery catheter 100. All other implementations known from pressure sensing PACs can of course be integrated in the alternative to the example shown here.

Inflation status determination unit 260 is configured to determine an inflation status of balloon 110 of PAC 100. To this end, for instance a pump connected to balloon port 112 corresponding to the port of PAC 100 communicating with the balloon 110 can be attached, which provides information indicative of the inflation status to inflation status determination unit 260. The inflation status determined by inflation status determination unit 260 comprises in this example the volume of liquid that has been injected into the balloon port 112 and preferentially comprises a derived suspected balloon status in percent of full balloon inflation. The inflation status determined by inflation status determination unit 260 can advantageously be used by the evaluation unit 250 for evaluating the hemodynamic data as will be described below.

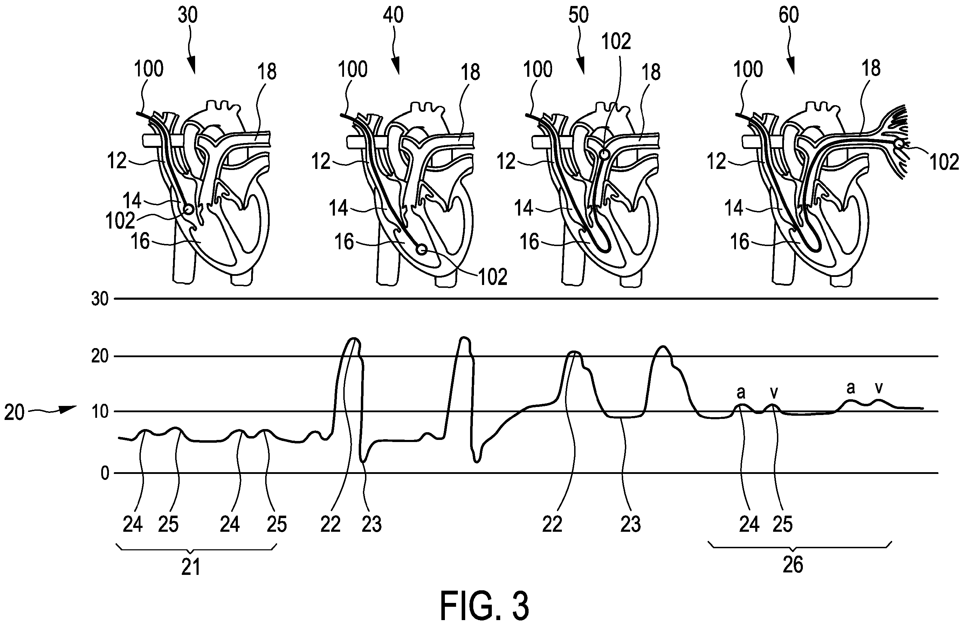

Evaluation unit 250 is configured to evaluate the hemodynamic data provided by hemodynamic data providing unit 240 in response to the segment determined by segment determination unit 230. Accordingly, evaluation unit 250 possesses additional information in addition to the hemodynamic data, namely a precise location corresponding to a segment of the heart, in which PAC 100 resides. This is based on the inventive finding that pressure time curves have typical shapes depending on in which chamber the catheter resides, i.e. depending on which segment PAC 100 is located in. Specific features in those hemodynamic data curves must thus be evaluated accordingly by evaluation unit 250. Typical hemodynamic pressure curves corresponding to specific segments or chambers of the heart 10 will be described schematically and exemplarily with reference to Fig. 3.

Fig. 3 schematically and exemplarily illustrates different positions 30, 40, 50 and 60 of distal tip 102 of PAC 100 within the heart 10 and a schematic pressure curve obtained at the respective positions in a lower diagram 20. Diagram 20 indicates a pressure on the vertical axis and both a timely and spatially evolution of the pressure sensed by distal tip 102 on a horizontal axis. The evolution on the horizontal axis would occur in case the catheter could transit from one of the respective positions 30, 40, 50 and 60 to a respective other of the positions without intermediate positions. In reality, the diagram 20 indicating a pressure curve over time would thus comprise a plurality of intermediate values.

First, with respect to position 30, distal tip 102 is located within the right atrium 14. Accordingly, segment determination unit 230 would identify the right atrium to be the current segment. In the corresponding portion of diagram 20, it can be seen that no significant pressure variations are present in the right atrium 14. Preferentially, evaluation unit 250 then, in case segment determination unit 230 determines the segment to be the right atrium, averages a mean pressure 21 over the time the catheter resides within the right atrium. Averaging in this example means the evaluation of average and standard deviation over all those R to R-peak (RR) intervals in which the catheter is present in the respective segment. In other examples, only some specific intervals of the persistence time are selected for generating the averages, as contemplated by the skilled person. Further, a- and v-waves can also be detected and averaged for the period, in which the PAC 100 resides in the right atrium 14, i.e. position 30.

Next, at position 40, catheter distal tip 102 has advanced into the right ventricle 16. In the right ventricle 16, high variations of pressure in one RR interval are present. Thus, in case evaluation unit 250 determines the current segment to be the right ventricle 16, a systolic pressure 22 and a diastolic pressure 23 can be detected and preferentially averaged. In the right ventricle, a generally expected systolic pressure would amount to 20 to 30 mm of mercury and a typically diastolic pressure would amount to 0 to 8 mm of mercury. Deviations from these values usually expected could be an indication for a pathological condition.

Next, in position 50, catheter distal tip 102 has advanced into the pulmonary artery 18. In this region, evaluation unit 250 can also be configured to automatically average and detect systolic 22 and diastolic 23 pressure values. In the pulmonary artery, different from the right ventricle, systolic values are expected to be in about the same magnitude, i.e. 20 to 30 mm of mercury, while diastolic pressures are expected to be higher, i.e. between 8 and 15 mm of mercury.

Next, in position 60, distal tip 102 is still located in a branch of the pulmonary artery 18, wherein in location 60 now balloon 110 of PAC 100 wedges the artery branch it recites in, so that a pulmonary wedge pressure can be acquired. The pulmonary artery wedge pressure is expected to be in a region of between 8 and 12 mm of mercury, i.e. higher than the right arterial pressure expected to be between 0 and 8 mm of mercury, but is devoid of variances such as systolic and diastolic variations. In contrast, only an a- and v-wave, indicated with 24 and 25, can be detected and averaged over the period, the PAC 100 resides with an inflated balloon 110. In addition, a mean wedge pressure 26 can be averaged over this period.

Returning to Fig. 2, PAC assistance of apparatus 200 further comprises the residence time evaluation unit 270 as mentioned above. Residence time evaluation unit 270 is configured to evaluate the time PAC 100 resides within a current segment. This can, for example, be done by monitoring the current segment and starting or stopping the residence time determination upon segment determination unit 230 determining a changed current segment. The residence time evaluation unit 270 then determines whether a hemodynamic data evaluated for a particular segment is enough, e.g. a minimum amount of time and/or heartbeats is evaluated or the standard deviation of the averaged measure drops below a certain threshold, and can provide an indication to the operator that the PAC 100 may be moved into the next position, i.e. the next segment, in case enough data has been collected. To this end, residence time evaluation unit 270 can be arranged to communicate with output means 13.

Chamber volume determination unit 290 is configured to determine a volume of a chamber of the heart, in particular of the right ventricle. To this end, in one example, chamber volume determination unit 290 is configured to receive MR images of at least the right ventricle of the heart and to calculate the volume of the chamber based on the received MR images.

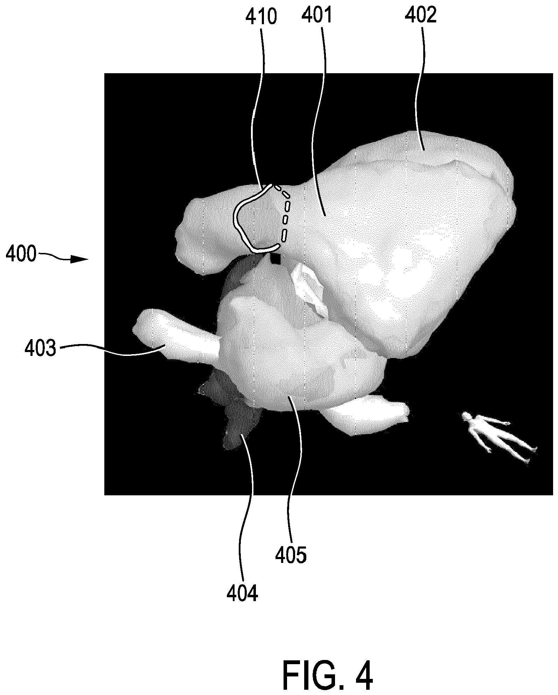

Display unit 280 is configured to generate a display for assisting a user, e.g. the operator of PAC 100, which then can be displayed on, for instance, output means 13. The generated display comprises a representation of the segmented model, wherein the segments are displayed with a respective different representation in response to the evaluation of evaluation unit 250. An example of differently represented segments will be described with reference to Fig. 4.

Fig. 4 schematically and exemplarily illustrates a display 400 of various segments 401, 402, 403, 404 and 405 of the segmented model provided by segmented model providing unit 220. The respective segments 401 to 405 are displayed with a respective different representation, wherein in this example the representation is changed to a specific representation accounting for differences in the hemodynamic data. For instance, healthy evaluation results can be represented in a different representation as unhealthy or pathologic segments. Further, segments that still lack measurements of hemodynamic data can have a different representation than those, of which already sufficient data has been evaluated. Further, mean or mean systolic, or mean diastolic pressures may be indicated per color. These are of course only examples, wherein other representations are of course contemplated.

Fig. 4 further indicates a cross section 410, in this example the pulmonary valve cross section, as an example of a cross section, for which a flow measurement can be carried out based on MR data. The use of cross section 410 for the present invention will be described with reference to an alternative of the functioning of chamber volume determination unit 290. As an alternative to the direct determination of the volume based on the MR image, chamber volume determination unit 290 can be configured to acquire a time resolved quantitative flow measurement both at the tricuspid valve cross section (not indicated in Fig. 4) and the pulmonary valve cross section 410.

Flow measurements of these cross sections are indicative of inflow and outflow of the right ventricle and the sum of time integrals of those flows is identical to the volume changes of the right ventricle overtime. In one example, segmented model providing unit 210 can be configured to further provide the locations of the required planes, e.g. the tricuspid valve and the pulmonary valve, to facilitate determination. It should be noted that this approach is only valid if there are no shunts or other obstructions present in the right ventricle.

Based on the determined chamber volume, pressure volume loop determination unit 300 is configured to measure a pressure volume loop in the right ventricle in a full scale examination. Preferentially, the determination of the pressure volume loop is triggered by the segment determination unit 230 determining the current segment to be the right ventricle. Expressed differently, the catheter 100 enters the right ventricle and then the pressure volume loop determination is started. The pressure volume loop acquisition comprises a simultaneous acquisition of pressure data, i.e. hemodynamic data, and volume data of the right ventricle obtained by the chamber volume determination unit 290.

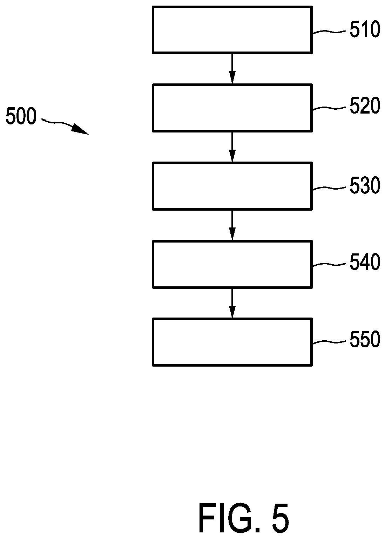

Finally, with reference to Fig. 5, a pulmonary artery catheterization assistance method 500 is schematically and exemplarily illustrated.

In first a step 510, location data of an actively tracked PAC 100 is provided. The location data is indicative of a position of the PAC.

In a step 520, a segmented model of the heart 10 of the subject 8 is provided, for instance by segmented model providing unit 220. Each segment corresponds to a predefined region, e.g. a chamber, of the heart 10.

In a step 530, a current segment of the plurality of segments is determined using the location data and the segmented model. The current segment corresponds to the location of the actively tracked PAC 100.

In a step 540, hemodynamic data corresponding to the location of the PAC 100 is provided, for instance, by hemodynamic data providing unit 240.

Finally, in a step 550, the hemodynamic data provided by hemodynamic data providing unit 240 is evaluated in reaction to the segment determined by, for instance, segment determination unit 230.

Other variations to the disclosed embodiments can be understood and effectuated by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or devices may fulfill the functions of several items recited in the claims. The mere fact that certain measures are recited in mutually different dependent claims does not indicate that a combination of these measures cannot be used to advantage.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

The present invention thus relates to an apparatus for assisting catheterization of a heart of a subject comprising a segmented model providing unit for providing a segmented model comprising a plurality of segments, wherein each segment corresponds to a predefined region of the heart, a segment determination unit for determining a current segment corresponding to the location of an actively tracked pulmonary artery catheter, a hemodynamic data providing unit for providing hemodynamic data corresponding to the location of the actively tracked pulmonary artery catheter, and an evaluation unit for evaluating the hemodynamic data in reaction to the determined segment. The pulmonary artery catheterization assistance apparatus as well as the corresponding pulmonary artery catheterization assistance method and computer program allow for an improved evaluation of hemodynamic data in pulmonary artery catheterization.

## Claims

1. A pulmonary artery catheterization assistance apparatus for assisting catheterization of a heart (10) of a subject (8), wherein the pulmonary artery catheterization assistance apparatus (200) comprises:
- a location data providing unit (210) for providing location data of an actively tracked pulmonary artery catheter (100), wherein the location data is indicative of a position of the actively tracked pulmonary artery catheter (100),
- a segmented model providing unit (220) for providing a segmented model of the heart (10) of the subject (8) comprising a plurality of segments (401, 402, 403, 404, 405), wherein each segment corresponds to a predefined region of the heart (10),
- a segment determination unit (230) for determining a current segment of the plurality of segments (401, 402, 403, 404, 405) of the segmented model using the location data, wherein the current segment corresponds to the location of the actively tracked pulmonary artery catheter (100),
- a hemodynamic data providing unit (240) for providing hemodynamic data corresponding to the location of the actively tracked pulmonary artery catheter (100), and
- an evaluation unit (250) for evaluating the hemodynamic data in reaction to the determined segment.

2. The pulmonary artery catheterization assistance apparatus as defined in claim 1, wherein the segment determination unit (230) is configured to determine a mean position of each segment of the segmented model and to determine the current segment as the segment with the corresponding mean position having the lowest distance to the location of the actively tracked pulmonary artery catheter (100).

3. The pulmonary artery catheterization assistance apparatus as defined in claim 1, wherein the hemodynamic data providing unit (240) is configured to provide an average of the hemodynamic data over the residence time of the actively tracked pulmonary artery catheter (100) in a segment.

4. The pulmonary artery catheterization assistance apparatus as defined in claim 1, wherein the segmented model providing unit (220) is configured to provide at least segments corresponding to the right atrium (14), the right ventricle (16) and the pulmonary arteries (18).

5. The pulmonary artery catheterization assistance apparatus as defined in claim 4, wherein the evaluation unit (250) is configured to at least one of:
- average a mean pressure in case the determined segment corresponds to the right atrium (14),
- detect and average an a and v wave height in case the determined segment corresponds to the right atrium (14), and
- average systolic and diastolic pressure in case the determined segment corresponds to the right ventricle (16) or the pulmonary artery (18).

6. The pulmonary artery catheterization assistance apparatus as defined in claim 1, wherein the pulmonary artery catheterization assistance apparatus (200) further comprises an inflation status determination unit (260) for determining an inflation status of the actively tracked pulmonary artery catheter (100). wherein the inflation status determination unit (260) is preferentially configured to determine the inflation status in reaction to a volume of liquid injected into a balloon port (112) of the actively tracked pulmonary artery catheter (100), and to derive the inflation status as a percentage of full balloon (110) inflation based on the volume of liquid injected.

7. The pulmonary artery catheterization assistance apparatus as defined in claim 6, wherein the inflation status determination unit (260) is configured to determine the balloon (110) to be inflated in case the inflation status exceeds a user defined threshold or maintains at a certain inflation status over a user defined minimum time.

8. The pulmonary artery catheterization assistance apparatus as defined in claim 6, wherein in case the inflation status determination unit (260) determines the balloon (110) to be inflated, the evaluation unit (250) is configured to at least one of:
- average a mean wedge pressure, and
- detect and average an a and v wave height.

9. The pulmonary artery catheterization assistance apparatus as defined in claim 1, wherein the pulmonary artery catheterization assistance apparatus (200) further comprises a display unit (280) for generating a display (400) for assisting a user, the generated display (400) comprising a representation of the segmented model, wherein the segments are displayed with a respective different representation in response to the evaluated hemodynamic data.

10. The pulmonary artery catheterization assistance apparatus as defined in claim 1, wherein the pulmonary artery catheterization assistance apparatus (200) further comprises
- a chamber volume determination unit (290) for determining a volume of a chamber of the heart based on provided MR image data, and
- a pressure-volume loop determination unit (300) for determining a pressure-volume loop by acquiring the volume of the chamber of the heart, determined from the chamber volume determination unit, and the hemodynamic data, provided by the hemodynamic data providing unit (240), at simultaneous times.

11. The pulmonary artery catheterization assistance apparatus as defined in claim 10, wherein the pressure-volume loop determination unit (300) is configured to initiate a pressure-volume loop acquisition in response to a change in the determined segment.

12. A pulmonary artery catheter for use with a pulmonary artery catheterization assistance apparatus (200) according to claim 1, the pulmonary artery catheter (100) comprising
- an inflatable balloon (110) arranged at a distal portion (102) of the catheter (100),
- a distal lumen including a distal lumen opening (142) arranged at a distal portion (102) of the catheter for providing hemodynamic data corresponding to the location of the distal lumen opening (142), and
- an active tracking component (120) for providing position data indicative of a position of the catheter (100) within a magnetic resonance imaging system, wherein the active tracking component (120) preferentially comprises at least one µ-coil and a magnetic resonance conditional wire along the catheter (100) being attached to the µ-coil.

13. A pulmonary artery catheterization system, the pulmonary artery catheterization system (1) comprising
- a magnetic resonance imaging apparatus (2) as defined in 11,
- a pulmonary artery catheter (100) according to claim 12, and
- a pulmonary artery catheterization assistance apparatus (200) as defined in claim 1.

14. A pulmonary artery catheterization assistance method for assisting catheterization of a heart (10) of a subject (8), wherein the pulmonary artery catheterization assistance method (500) comprises:
- providing (510) location data of an actively tracked pulmonary artery catheter (100), wherein the location data is indicative of a position of the actively tracked pulmonary artery catheter (100),
- providing (520) a segmented model of the heart (10) of the subject (8) comprising a plurality of segments, wherein each segment corresponds to a predefined region of the heart (10),
- determining (530) a current segment of the plurality of segments of the segmented model using the location data, wherein the current segment corresponds to the location of the actively tracked pulmonary artery catheter (100),
- providing (540) hemodynamic data corresponding to the location of the actively tracked pulmonary artery catheter (100), and
- evaluating (550) the hemodynamic data in reaction to the determined segment.

15. A computer program for assisting catheterization of a heart (10) of a subject (8), the computer program comprising program code means for causing a pulmonary artery catheterization system (1) as defined in claim 13 to carry out the steps of the pulmonary artery catheterization assistance method (500) as defined in claim 14, when the computer program is run on a processing unit of the pulmonary artery catheterization system (1).
